# EUROPÄISCHE PATENTSCHRIFT

(11) **EP 2 868 334 B1**
(45) Veröffentlichungstag und Bekanntmachung des Hinweises auf die Patenterteilung: **11.01.2017**
(21) Anmeldenummer: 13005214.5
(22) Anmeldetag: 05.11.2013
(51) Int. Cl.: A61M 3/02, B05B 11/00, F16K 31/126, B05B 9/08, F16K 3/26, B05B 1/30, A61H 35/04

(54) **NASENDUSCHE**
NASAL SPRAY
DOUCHE NASALE

(43) Veröffentlichungstag der Anmeldung: 06.05.2015
(73) Patentinhaber: Gerber, Benedict, 8598 Bottighofen (CH)
(72) Erfinder: Gerber, Benedict, 8598 Bottighofen (CH)
(74) Vertreter: Schaad, Balass, Menzl & Partner AG

(56) Entgegenhaltungen:
- KR-A- 20110 056 462

## Beschreibung

Die vorliegende Erfindung betrifft eine Nasendusche nach dem Oberbegriff des Patentanspruchs 1.

Aus KR 2011 0056462 A ist eine Nasendusche bekannt, die einen Behälterabschnitt mit einer Spülflüssigkeit und einen Rohrabschnitt mit einem Venturi-Rohrabschnitt zum Sprühen der Spülflüssigkeit aufweist. Der Grundkörper des Rohrabschnitts ist C-förmig gebogen und der Venturi-Rohrabschnitt ist T-förmig am zentralen unteren Ende des C-förmigen Rohrabschnitts angeformt. Wenn ein Benutzer Luft in ein Ende des Venturi-Rohrabschnitts bläst wird ein Unterdruck erzeugt, wodurch die Spülflüssigkeit aus dem Behälterabschnitt angesaugt und über den Rohrabschnitt in Richtung zum Nasenloch des Benutzers transportiert wird, um die Nase des Benutzers mit der Spülflüssigkeit zu spülen.

Dokument EP 2 036 527 A1 offenbart eine Nasendusche mit einem elastischen Behälter zur Aufnahme von Spülflüssigkeit, wobei der Behälter eine Spülöffnung und eine mit einem Finger verschliessbare Belüftungsöffnung aufweist. Die Belüftungsöffnung ist mit einem Rückschlagventil versehen, welches im Gebrauch bei drucklosem Behälter geöffnet ist und ein Austreten von Spülflüssigkeit aus der Spülöffnung zulässt. Durch rasches Zusammendrücken des Behälters kann das Rückschlagventil geschlossen werden, wodurch ein Austreten von Spülflüssigkeit unter Druck aus der Spülöffnung möglich ist.

Nachteilig an der bekannten Nasendusche ist, dass nach dem Befüllen des elastischen Behälters mit Spülflüssigkeit die Nasendusche im Gebrauch stets in der Hand gehalten werden muss, da wegen der geöffneten Belüftungsöffnung die Spülflüssigkeit andernfalls ununterbrochen aus der Spülöffnung ausläuft, was einen sehr unzweckmässigen Gebrauch der bekannten Nasendusche mit sich bringt. Das einzige Kontrollsystem, um das Abfliessen bei der bekannten Nasendusche im Gebrauch zu unterbrechen, ist das manuelle Verschliessen der Belüftungsöffnung mit dem Finger, wodurch zwar das Abfliessen gestoppt wird, hingegen ist ein kontrolliertes Abfliessen der Spülflüssigkeit aus dem Behälter der Nasendusche auf diese Art und Weise nicht möglich.

Des Weiteren hat die bekannte Nasendusche üblicherweise den weiteren Nachteil, dass die Spülflüssigkeit, welche über ein Nasenloch der Nase des Benutzers eintritt, nicht nur aus dem anderen Nasenloch wieder austritt, sondern aufgrund der Verbindung des Nasenraums mit dem Mund-Rachenraum auch in den Mund-Rachenraum abfliessen kann, wodurch ein Verschlucken der Spülflüssigkeit die Folge sein kann. Dies ist jedoch nicht wünschenswert, da die Spülflüssigkeit in der Regel ausschliesslich dazu dient, den Nasenraum des Benutzers zu spülen. Zudem kann die Spülflüssigkeit beispielsweise auch einen unerwünschten Brechreiz beim Benutzer während des Gebrauchs der bekannten Nasendusche auslösen.

Weiter ist auch ein passiver Übertritt oder ein aktives Aspirieren der Spülflüssigkeit in den Kehlkopf und die unteren Atemwege mit der bekannten Nasendusche möglich, wobei dies für den Benutzer als unangenehm empfunden werden kann oder unter gegebenen Umständen für den Benutzer sogar gefährlich sein kann.

Es ist somit eine Aufgabe der vorliegenden Erfindung, die bekannte Nasendusche derart weiterzubilden, dass diese einfacher zu handhaben ist, ein kontrolliertes Abfliessen der Spülflüssigkeit aus dem Behälter ermöglicht und zudem das Abfliessen von Spülflüssigkeit in den Mund-Rachenraum verhindert ist.

Diese Aufgabe wird erfindungsgemäss durch eine Nasendusche mit den Merkmalen des Patentanspruchs 1 gelöst.

Die Nasendusche gemäss dem Patentanspruch 1 weist ein Ventil, einen Behälterteil und einen Kappenteil auf, wobei der Behälterteil zur Aufnahme von Spülflüssigkeit dient und den Kappenteil trägt. Der Kappenteil hat einen Nasenansatz mit einer über einen Ausströmungskanal mit einem Inneren des Behälterteils strömungsverbundenen Austrittsöffnung für die Spülflüssigkeit, wobei der Nasenansatz dazu bestimmt ist an ein Nasenloch des Benutzers angesetzt zu werden.

Erfindungsgemäss ist dem Behälterteil oder dem Kappenteil ein Mundstück zur Steuerung des Ventils zugeordnet, wobei das Ventil in einer Ruhestellung geschlossen und dadurch das Ausfliessen von Spülflüssigkeit durch den Nasenansatz unterbunden ist. Durch Beaufschlagung des Ventils mit Atemluft, indem der Benutzer in das Mundstück Atemluft einbläst, ist das Ventil in eine Offenstellung verbringbar und dadurch fliesst Spülflüssigkeit durch den Nasenansatz aus, wobei zum Ausfliessen Atemluft oder Umgebungsluft in den Behälterteil einströmt.

Ein wesentlicher Vorteil bei der vorliegenden Erfindung ist die Beaufschlagung des Ventils mit Atemluft über das dem Behälterteil oder Kappenteil zugeordnete Mundstück, um das Ventil in die Offenstellung zu verbringen. Auf diese Art und Weise ist es möglich, entweder Atemluft oder Umgebungsluft in den Behälterteil einzubringen, wodurch ein Druckunterschied im Behälterteil erzeugt wird. Aufgrund dieses Effektes ist in vorteilhafter Weise ein kontrolliertes Abfliessen der Spülflüssigkeit aus dem Behälterteil der erfindungsgemässen Nasendusche in Abhängigkeit vom eingeblasenen Atemdruck gewährleistet.

Im Unterschied zur bekannten Nasendusche ist somit ein kontrolliertes Abfliessen der Spülflüssigkeit ohne manuelle Betätigung aus dem Behälterteil möglich, so dass die freie verfügbare Hand dem Benutzer die Handhabung der erfindungsgemässen Nasendusche erheblich vereinfacht. Es muss lediglich eine Hand verwendet werden, um den Kappenteil zu halten, und die in das Mundstück kontrolliert eingeblasene Atemluft erlaubt das Abfliessen der Spülflüssigkeit aus dem Behälterteil in Richtung zur Austrittsöffnung des Nasenansatzes mit einer kontrollierten Durchflussrate.

Ebenfalls von Vorteil, im Vergleich zur bekannten Nasendusche, ist die uneingeschränkte Verwendung von Behälterteilen für die Spülflüssigkeit, sei es mit elastischen oder starren Eigenschaften. Aufgrund des kontrollierten Einblasens der Atemluft durch das Mundstück kann je nach gewünschter Durchflussrate mit einem höheren oder auch mit einem niedrigeren Atemdruck die Spülflüssigkeit aus dem Behälterteil in Richtung zur Austrittsöffnung abfliessen. Hierbei muss jedoch der Behälterteil nicht zusammengedrückt werden, wie dies bei der bekannten Nasendusche der Fall ist, um einen höheren Druck im Inneren des Behälters zu erzeugen.

Ein weiterer wesentlicher Vorteil bei der vorliegenden Erfindung ist die funktionale Wirkung des Einblasens von Atemluft in das Mundstück. Da der Benutzer im Gebrauch der erfindungsgemässen Nasendusche beim Einblasen der Atemluft in das Mundstück mit seinen Lippen das Mundstück dichtend umschliesst, wird im Mundraum ein Druckanstieg durch aktives Ausatmen verursacht. Dies erfolgt insbesondere dadurch, dass das Gaumensegel, welches eine Muskelplatte ist, den Nasenraum unwillkürlich abschliesst, wodurch einerseits Druck auf das Mundstück und nachgeschaltete Volumina übertragen wird und andererseits wird die durch den Nasenansatz druckbedingt in ein Nasenloch geleitete Spülflüssigkeit zwingend im Nasenrachen um die hintere Kante der Nasenscheidewand herumgeführt und über das gegenseitige Nasenloch wieder abgeleitet. Folglich ist ein Übertritt der Spülflüssigkeit in die unter dem Gaumen liegenden Atemwege unterbunden und entsprechend ist ein Verschlucken, Eindringen in die unteren Atemwege oder Aspiration im Vergleich zur bekannten Nasendusche nicht mehr möglich.

Bevorzugt sind der Behälterteil als eigenständiges Bauteil und der Kappenteil als eigenständiges Bauteil ausgebildet und diese sind vorzugsweise trennbar aneinander befestigt. Dies hat den Vorteil, dass bei abgetrenntem Kappenteil der Behälterteil mit Spülflüssigkeit gefüllt werden kann.

Weiter bevorzugt sind der Kappenteil und der Behälterteil mittels einer Schraubverbindung oder eines Bajonettverschlusses aneinander befestigt. Dies ermöglicht eine sehr einfache und trennbare Verbindung ohne dabei den Behälterteil oder den Kappenteil zu beschädigen. Des Weiteren kann der Behälterteil der Nasendusche problemlos wieder mit der Spülflüssigkeit aufgefüllt werden.

Für den Fall der Schraubverbindung als Befestigung zwischen dem eigenständigen Kappenteil und dem eigenständigen Behälterteil ist es von Vorteil, wenn die Schraubverbindung derart ausgebildet ist, dass bereits nach geringfügiger Verschraubung zwischen dem eigenständigen Kappenteil und dem eigenständigen Behälterteil eine feste Verbindung besteht und zugleich eine eindeutig definierte Position des eigenständigen Kappenteils bezüglich des eigenständigen Behälterteils einstellbar ist. Dies ist insbesondere dann von Vorteil, wenn der eigenständige Behälterteil zu einer Achse asymmetrisch oder ebenensymmetrisch ausgebildet ist und entsprechend vor dem Gesicht des Benutzers ausgerichtet sein muss, sobald der am Kappenteil zugeordnete Nasenansatz dem Benutzer zugewandt ist.

Bei eigenständigem Kappenteil ist dieses vorzugsweise schalenartig ausgeformt und dient der verbesserten Stabilität bei der Verbindung mit dem Behälterteil. Auf diese Art und Weise kann überdies ein Abschnitt des Behälterteils von der schalenartigen Ausformung umfasst werden. Durch geeignete Formgebung des Kappenteils wird je nach Bedarf ein guter Zugang zum Ventilglied zwecks vereinfachten Austauschs desselben gewährleistet.

Vorzugsweise kann das eigenständige Kappenteil in abgeschraubtem Zustand aufrecht gelagert werden, wobei sich der Nasenansatz in der Luft befindet und entsprechend vor Verunreinigung geschützt ist, und der Ausströmungskanal des Nasenansatzes weist einen Winkel ausserhalb der Horizontalen auf, so dass eine Restentleerung des Ausströmungskanals und eine Belüftung während der Lagerung spontan erfolgen können.

Ebenso ist es auch denkbar, dass das eigenständige Kappenteil und das eigenständige Behälterteil mittels eines Presssitzes aneinander befestigt sind.

Es ist auch möglich den Behälterteil einstückig mit dem Kappenteil auszubilden. Dies erlaubt eine kompakte Bauweise der erfindungsgemässen Nasendusche mit ergonomischen Eigenschaften für die Anwendung.

Weiter bevorzugt weist der Behälterteil eine Nachfüllöffnung auf, welche mittels eines Deckels verschliessbar ist. Der Deckel ist vorzugsweise auf der dem Kappenteil abgewandten Seite der Nasendusche angeordnet, um das Befüllen des Behälterteils mit Spülflüssigkeit zu vereinfachen.

Bevorzugt verschliesst das Ventil in Ruhestellung einen vom Mundstück zum Behälterteil führenden Einblasluftkanal, wodurch keine Atemluft oder Umgebungsluft in den Behälterteil gelangt und das Abfliessen von Spülflüssigkeit aus dem Behälterteil in Richtung zur Austrittsöffnung unterbunden ist und das Ventil bei Beaufschlagung mit Atemluft in Offenstellung verbringbar ist, so dass die Atemluft über den Einblasluftkanal oder Umgebungsluft in den Behälterteil gelangt und die Spülflüssigkeit vom Behälterteil in Richtung zur Austrittsöffnung abfliessen kann. Bei dieser Anordnung des Ventils kann durch kontrolliertes Einblasen der Atemluft durch das Mundstück eine definierte Luftmenge in den Behälterteil eingeführt werden, um die Durchflussrate der Spülflüssigkeit aus dem Behälterteil zu kontrollieren.

Für den Fall, dass das Ventil mit der Spülflüssigkeit in Kontakt steht, d.h. das Ventil sich im Inneren des Behälterteils befindet oder in der Aussenwand des Behälterteils eingebracht ist, besteht die Aufgabe des Ventils darin, zu verhindern, dass Spülflüssigkeit aus dem Behälterteil über den Einblasluftkanal zum Mundstück zurückfliessen kann. Folglich wirkt der hydrostatische Druck der Spülflüssigkeit dem Umgebungsdruck entgegen und bei Beaufschlagung des Ventils mit Atemluft muss erst die Schliesskraft des Ventils überwunden werden, bevor Atemluft in den Behälterteil gelangen kann. Erst wenn keine Atemluft mehr über das Mundstück in den Behälterteil eingeblasen wird, gelangt das Ventil wieder in seine Ruhestellung zurück, so dass der Zugang der Spülflüssigkeit zum Mundstück und zur Austrittsöffnung wieder verschlossen ist.

Alternativ ist es auch denkbar, dass das Ventil in einem Einlasskanal zwischen der Umgebung und der Spülflüssigkeit angeordnet ist, so dass bei Beaufschlagung des Ventils mit Atemluft das Ventil in die Offenstellung verbringbar ist und dabei den Strömungspfad von der Umgebung in das Innere des Behälterteils zulässt, wodurch Umgebungsluft in den Behälterteil gelangen kann und dort das Abfliessen der Spülflüssigkeit vom Inneren des Behälterteils in Richtung zur Austrittsöffnung des Nasenansatzes ermöglicht.

Auch ist es denkbar, dass im Einblasluftkanal zwischen dem Mundstück und dem Ventil im Inneren des Behälterteils ein Pilotventil angeordnet ist, welches mittels Atemluft gesteuert wird und das Pilotventil wiederum steuert das Ventil im Inneren des Behälterteils.

Bevorzugt weist das Ventil einen vorzugsweise an den Kappenteil angeformten vorzugsweise ringförmigen Ventilsitz und ein mit diesem zusammenwirkendes vorzugsweise tellerförmiges Ventilglied auf. An dieser Stelle sei erwähnt, dass es sich bei diesem Ventil um eine spezifische Lösung handelt, um das Eintreten von Spülflüssigkeit aus dem Behälterteil in den Einblasluftkanal zu unterbinden, wobei selbstverständlich auch andere technische Lösungen in Frage kommen können.

Weiter bevorzugt weist ein Steg einen vorzugsweise wenigstens annährend eiförmigen oder kugelförmig verdickten Kopf auf, wobei das Ventilglied vorzugsweise unter Vorspannung am Ventilsitz anliegt sowie membranartig, elastisch verformbar ausgebildet ist. Das Ventilglied weist bevorzugt zentrisch ein Durchgangsloch auf, ist bei der Montage mit dem Durchgangsloch über den Kopf ziehbar und hintergreift im montierten Zustand den Kopf. Da das Ventilglied membranartig, elastisch verformbar ist, kann auf einfache Art und Weise das Ventilglied schnell und auf einfache Art und Weise ausgetauscht werden. Dies ist insbesondere aus hygienischen Gründen von Vorteil.

Der Kopf hält das Durchgangsloch des montierten Ventilglieds in Position und der radial äussere Rand des tellerförmigen Ventilglieds hebt sich bei Überwindung der Schliesskraft vom Ventilsitz ab, um die Atemluft in das Innere des Behälterteils eintreten zu lassen. Dieser Vorgang verursacht eine Druckänderung im Behälterteil, wodurch das Abfliessen der Spülflüssigkeit aus dem Behälterteil in Richtung zur Austrittsöffnung ermöglicht wird.

Da das Ventilglied vorzugsweise unter Vorspannung am Ventilsitz anliegt und bei Einblasen der Atemluft in das Mundstück sich das Ventilglied erst bei Überschreiten der Schliesskraft vom Ventilsitz abhebt, gelangt bei Unterbrechung des Einblasens von Atemluft in das Mundstück das Ventilglied, aufgrund der vorgespannten Rückstellkraft, sehr schnell wieder zurück in die geschlossene Ruhestellung. Auf diese Art und Weise ist gesichert, dass in der Ruhestellung keine Spülflüssigkeit aus dem Behälterteil über den Einblasluftkanal zum Mundstück gelangen kann.

Bevorzugt weist das Mundstück ein elastisches Material auf oder ist aus einem solchen hergestellt, so dass unterschiedliche Abstände zwischen Mund und Nase des Benutzers bei der Anwendung, beziehungsweise durch Verbiegen des Mundstücks, kompensierbar sind.

Bevorzugt ist das Mundstück austauschbar und liegt vorzugsweise in verschiedenen Dimensionen für physiognomisch unterschiedliche Anwendergruppen vor.

Bevorzugt ist das Mundstück auswechselbar in eine Aufnahme des Kappenteils einsteckbar oder auf einen Ansatz des Kappenteils aufsteckbar, wobei das Mundstück vorzugsweise gerade oder abgewinkelt ausgebildet ist.

Bei einer weiteren Ausführungsform der vorliegenden Erfindung verschliesst das Ventil in Ruhestellung den Ausströmungskanal und gibt in Offenstellung den Ausströmungskanal frei. Durch Druckbeaufschlagung mit Atemluft aus dem Mundstück kann das Ventil in die Offenstellung verbracht werden, um das kontrollierte Abfliessen der Spülflüssigkeit aus dem Behälterteil zu ermöglichen.

Bevorzugt weist das Ventil einen Durchgang quer zum Ausströmungskanal als Ventilsitz auf, welcher mit einem als Verschlussstift ausgebildeten und mit einem Betätigungsorgan zusammenwirkenden Ventilglied derart zusammenwirkt, dass in Ruhestellung der Verschlussstift den Ausströmungskanal verschliesst und in Offenstellung, bei Beaufschlagung mit Atemluft, das Betätigungsorgan den Verschlussstift derart verschiebt, dass der Durchlass den Ausströmungskanal freigibt.

Bevorzugt weist der Behälterteil eine Belüftungsöffnung auf und ist vorzugsweise mit einem Rückschlagventil versehen, welches das Austreten von Spülflüssigkeit aus dem Behälterteil durch die Belüftungsöffnung verhindert und das Einströmen von Umgebungsluft durch die Belüftungsöffnung in den Behälterteil zulässt.

Bevorzugt kann der Kappenteil ein Ventil, wie oben beschrieben, aufweisen, um das Einblasen von Atemluft in den Behälterteil zu gewährleisten, und ein weiteres Ventil kann dazu dienen, einen Durchlass in einem Verschlussstift mittels eines Betätigungsorgans zum Ausströmungskanal des Nasenansatzes freizugeben. Entsprechend weist das Mundstück vorzugsweise einen Abzweiger auf, welcher einerseits das Einblasen der Atemluft in den Behälterteil und gleichzeitig das Einblasen von Atemluft zum Betätigungsorgan ermöglicht.

Bevorzugt ist die Wirkung des Ventils auch durch einen im Behälterteil angeordneten Ballon erzielbar, wobei bei Beaufschlagung von Atemluft durch Einblasen in das Mundstück, die Atemluft im Einblasluftkanal den Ballon derart aufbläht, dass dieser die Spülflüssigkeit im Behälterteil derart verdrängt, dass die Spülflüssigkeit vom Behälterteil in Richtung zur Austrittsöffnung des Nasenansatzes abfliessen kann.

Bei einer weiteren Ausführungsform ist es denkbar, dass der Nasenansatz nicht mit dem Kappenteil integral ausgebildet ist, sondern der Nasenansatz ist ein eigenständiges, geradliniges Bauteil, welches in einer sacklochförmigen Aufnahme in seiner Längsrichtung verschiebbar gelagert ist. Die Aufnahme ist durch den Kappenteil und einen daran integral angeformten Fortsatz ausgebildet. Der Ausströmungskanal weist innen, kreuzweise radiale erste Durchlässe durch den Nasenansatz auf, die mit dem Ausströmungskanal verbunden sind und weist weiter kreuzweise radiale zweite Durchlässe auf, die vom Ausströmungskanal getrennt sind. Eine zwischen dem Fortsatz und dem Nasenansatz wirkende Feder drängt den Nasenansatz in eine Ruhestellung, wobei die ersten und zweiten Durchlässe durch den Kappenteil verschlossen sind und in einer Offenstellung geben einerseits die zweiten Durchlässe den Verbindungskanal frei und stellt andererseits für die ersten Durchlässe die Strömungsverbindung zwischen dem Inneren des Behälterteils und dem Ausströmungskanal des Nasenansatzes her.

Weitere Vorteile und Eigenschaften der erfindungsgemässen Nasendusche gehen aus der nachstehenden Beschreibung von Ausführungsbeispielen hervor, welche anhand der Zeichnung erläutert werden.

Es zeigen rein schematisch:
- Fig. 1: einen Längsschnitt einer erfindungsgemässen Nasendusche mit einem eigenständigen Behälterteil zur Aufnahme von Spülflüssigkeit, einem auf dem Behälterteil aufgeschraubten eigenständigen Kappenteil mit einem Nasenansatz und einem zugeordneten Mundstück sowie einem Ventil, welches in Ruhestellung das Abfliessen von Spülflüssigkeit durch das Mundstück verhindert;
- Fig. 2: einen Längsschnitt der erfindungsgemässen Nasendusche gemäss Fig. 1 in Zusammenschau mit einem Längsschnitt des Nasen-Rachenraums eines Benutzers;
- Fig. 3: einen Längsschnitt einer erfindungsgemässen Nasendusche mit einem Behälterteil und einem Kappenteil, die zusammen einstückig ausgebildet sind, wobei der Behälterteil zur Aufnahme von Spülflüssigkeit dient, und der Kappenteil mit einem Nasenansatz und einem zugeordneten Mundstück sowie einem Ventil versehen ist, so dass das Ventil in Ruhestellung das Abfliessen von Spülflüssigkeit durch das Mundstück verhindert;
- Fig. 4: einen Längsschnitt durch einen Nasenansatz in Offenstellung, wobei der Nasenansatz in eine sacklochförmige Aufnahme in Längsrichtung eingefahren;
- Fig. 5: einen Längsschnitt durch den Nasenansatz in Ruhestellung, wobei der Nasenansatz in der sacklochförmigen Aufnahme in Längsrichtung ausgefahren ist;
- Fig. 6: eine Draufsicht auf eine Ausführungsform, bei der ein Ventil einen Ausströmungskanal im Nasenansatz verschliesst;
- Fig. 7: einen Längsschnitt durch den Ausströmungskanal entlang der Linie A-A gemäss Fig. 6 in Ruhestellung;
- Fig. 8: einen Längsschnitt durch einen Ansatz von einem Mundstück und ein Betätigungsorgan für ein Ventil entlang der Linie B-B gemäss Fig. 6 in Ruhestellung; und
- Fig. 9: einen Längsschnitt durch den Ausströmungskanal gemäss Fig. 6 bei Druckbeaufschlagung mit Atemluft aus dem Mundstück in Offenstellung.

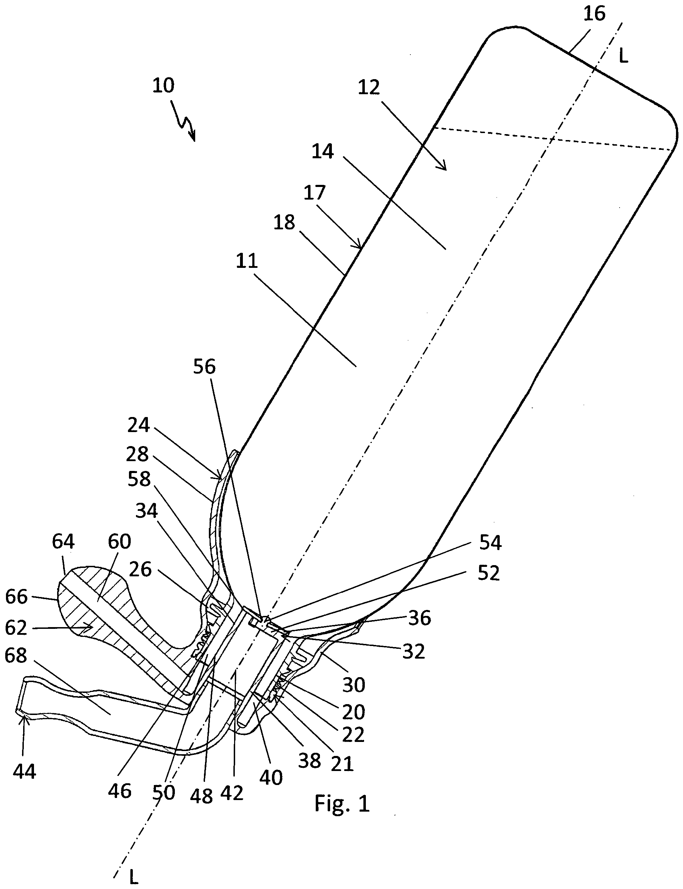

Ein in Fig. 1 gezeigtes erstes Ausführungsbeispiel gemäss einer erfindugnsgemässen Nasendusche 10 ist in Gebrauchsstellung (Überkopflage) gezeigt, also um 180° gekippt.

Die Nasendusche 10 weist im Inneren 11 von einem eigenständigen Behälterteil 12 eine Spülflüssigkeit 14 auf und trägt einen eigenständigen Kappenteil 24. Bei diesem Ausführungsbeispiel sind der Behälterteil 12 und der Kappenteil 24 eigenständige Bauteile, die mittels einer Schraubverbindung 21 aneinander trennbar befestigt sind.

Der Behälterteil 12 kann je nach Anwendung beispielsweise ein Füllvolumen von ca. 100 ml bis ca. 500 ml aufweisen und ist beispielsweise eine Flasche 12' mit einem Flaschenboden 16 und einem an einen Flaschenbauch 18 anschliessenden verjüngenden Flaschenhals 20.

Das maximale Füllvolumen des Behälterteils 12 richtet sich unter anderem an die praxisorientierte Handhabbarkeit der erfindungsgemässen Nasendusche 10, d.h. das Füllvolumen sollte nicht so hoch sein, dass der Benutzer die Nasendusche 10 nur noch mit Mühe mit einer Hand halten kann.

Der Behälterteil 12 kann eine allgemein bekannte Flasche oder jede Art von Behälterteil 12 sein, welche befähigt ist die Spülflüssigkeit 14 in ihrem Inneren 11 aufzunehmen.

Für die Schraubverbindung 21 zwischen dem Kappenteil 24 und dem Behälterteil 12 ist an der Aussenseite des Flaschenhalses 20 ein Aussengewinde 22 angebracht.

Der Behälterteil 12 kann aus einem harten Material ebenso wie aus einem elastischen Material hergestellt sein. Die Materialeigenschaften des Behälterteils 12 haben keinen Einfluss auf die Funktionalität der erfindungsgemässen Nasendusche 10. Dies im Gegensatz zur bekannten Nasendusche, wie in der Einleitung ausgeführt, bei der der Behälter elastisch ausgebildet sein muss, um einen höheren Druck durch ein manuelles Zusammendrücken des Behälters zu erzeugen.

Der eigenständige Kappenteil 24 ist für die Schraubverbindung 21 mit einem Innengewinde 26 versehen, und gemäss Fig. 1 auf das Aussengewinde 22 des Behälterteils 12 aufgeschraubt gezeigt.

In diesem Zusammenhang sei erwähnt, dass es auch möglich ist, dass der Kappenteil 24 ein zu herkömmlichen Flaschen, wie beispielsweise PET-Flaschen, nicht kompatibles Innengewinde aufweist, so dass ein spezifischer Behälterteil mit einem entsprechenden Aussengewinde bei der Nasendusche zu verwenden ist.

Als eigenständiger Kappenteil 24 hat dieser vorzugsweise einen schalenartigen Abschnitt 28 und dient im eingebauten Zustand als mechanische Stütze für den Behälterteil 12 im Bereich des Übergangs vom Flaschenbauch 18 zum verjüngten Flaschenhals 20. Der schalenartige Abschnitt 28 ist in diesem Fall mit dem Kappenteil 24 integral ausgebildet.

In den schalenartigen Abschnitt 28 ist vorzugsweise wenigstens ein tiefer Ausschnitt 30 eingebracht, der es dem Benutzer der Nasendusche 10 erlaubt ein Ventil 32 aus hygienischen Gründen zu warten.

Das Ventil 32 weist einen an den Kappenteil 24 angeformten, ringförmigen Ventilsitz 34 und ein mit diesem zusammenwirkendes, tellerförmiges Ventilglied 36 auf.

Der ringförmige Ventilsitz 34 ist gebildet durch das freie Ende einer zur mit der Behälterachse zusammenfallenden Längsachse L koaxialen Einstülpung 38 des Kappenteils 24 in der Durchlassöffnung 40 des Flaschenhalses 20. Die Einstülpung 38 ist hohlzylinderförmig und bildet eine Aufnahme 42 für ein rohrartiges, abgewinkeltes Mundstück 44 aus. Zwischen der Aussenseite 46 der hohlzylinderförmigen Einstülpung 38 und der Innenseite 48 des hohlzylinderförmigen Flaschenhalses 20 ergibt sich ein rohrförmiger Durchgang 50 für die Spülflüssigkeit 14.

Die Einstülpung 38 des Kappenteils 24 ist zum Flaschenboden 16 hin geöffnet und bildet den ringförmigen Ventilsitz 24 aus. Die Einstülpung 38 weist benachbart zum Ventilsitz 24 einen Steg 52 nach radial innen mit einem zentral zum Ventilsitz 24 angeordneten, kugelförmig verdickten Kopf 54 auf. Das Ventilglied 36 ist membranartig und elastisch verformbar ausgebildet und hat zentrisch ein Durchgangsloch 56.

Bei der Montage des Ventils 32 ist das Ventilglied 36 mit dem Durchgangsloch 56 über den Kopf 54 ziehbar und im montierten Zustand hintergreift das elastisch ausgebildete Ventilglied 36 den Kopf 54. Hierbei liegt das Ventilglied 36 unter Vorspannung am Ventilsitz 34 an, da der Hintergriff bezüglich des Ventilsitzes 24 zurück versetzt ist. Im Ruhezustand des Ventilgliedes 36, d.h. bei geschlossenem Ventil 32, ist das Ausströmen von Spülflüssigkeit 14 verhindert.

Beim dem Flaschenboden 16 abgewandten Ende 58 des dort in axialer Richtung geschlossenen Durchgangs 50 für die Spülflüssigkeit 14 verbindet von der Seite her ein Ausströmungskanal 60 eines Nasenansatzes 62 eine Austrittsöffnung 64 mit dem Durchgang 50. Der Nasenansatz 62 ist in vorliegendem Ausführungsbeispiel einstückig an den Kappenteil 24 angeformt und steht seitlich vor, wobei der Ausströmungskanal 60 des Nasenansatzes 62 und der ringförmige Durchgang 50 für die Spülflüssigkeit 14 strömungsverbunden sind.

Der Ausströmungskanal 60 des Nasenansatzes 62 endet in der endseitigen Austrittsöffnung 64 des Nasenansatzes 62, wobei der Ausströmungskanal 60 in vorliegendem Ausführungsbeispiel in Richtung des Flaschenbodens 16 abgewinkelt angeordnet ist, sodass der Winkel zwischen der Längsachse L und der Längseinstellung des Nasenansatzes 62 kleiner als 90° ist.

In diesem Zusammenhang sei erwähnt, dass der Ausströmungskanal 60 auch parallel zum Flaschenboden 16 und somit rechtwinklig zur Längsachse L des Behälterteils 12 angeordnet sein könnte.

Es ist auch denkbar, aber nicht gezeigt, dass der Behälterteil 12 zur Längsachse L asymmetrisch oder ebenensymmetrisch ausgebildet ist, so dass der Ausströmungskanal 60 stumpfwinklig zur Längsachse L des Behälterteils 12 angeordnet sein könnte.

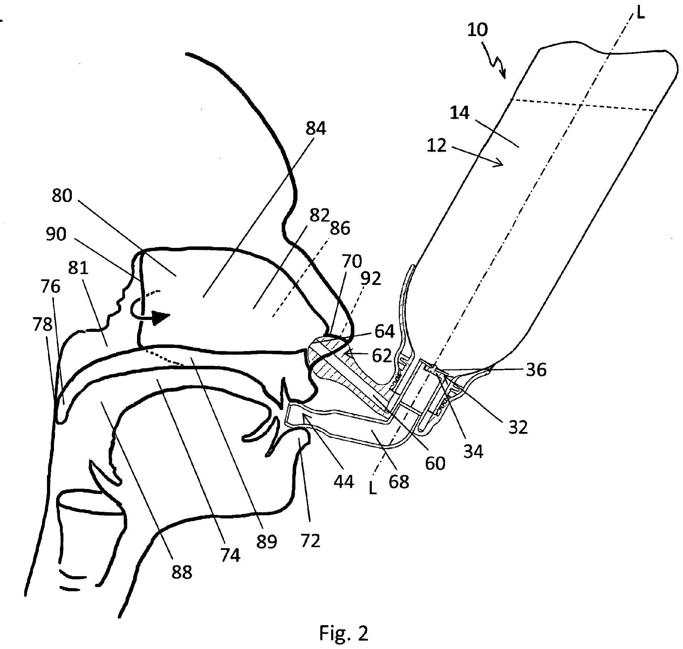

Ein nasenseitiger Endbereich 66 des Nasenansatzes 62 ist olivenförmig verdickt ausgebildet und ist dazu geeignet, an ein Nasenloch des Benutzers (siehe Fig. 2) angesetzt zu werden. In diesem Zusammenhang sei erwähnt, dass der nasenseitige Endbereich 66 je nach anatomischer Form des Nasenloches des Benutzers verschieden ausgeformt sein kann, um einen optimalen Sitz des nasenseitigen Endbereichs 66 des Nasenansatzes 62 mit der Nase des Benutzers zu ermöglichen. Hierbei ist unteranderem zu berücksichtigen, dass im Gebrauch der Nasendusche 10 keine Spülflüssigkeit 14 zwischen dem Nasenloch des Benutzers und dem nasenseitigen Endbereich 66 des Nasenansatzes 62 austreten sollte und entsprechend eine ausreichende Abdichtung gewährleistet sein sollte.

Der Nasenansatz 62 ist vorzugsweise starr gegenüber dem Behälterteil 12 montiert, um ein einwandfreies Spülen der Nase zu ermöglichen und dabei ein Verrutschen des nasenseitigen Endbereichs 66 des Nasenansatzes 62 in der Nase zu verhindern.

Das Mundstück 44 ist im vorliegenden Ausführungsbeispiel drehbar in der hohlzylinderförmigen Aufnahme 42 eingesteckt.

Das Mundstück 44 ist dazu bestimmt, vom Benutzer in den Mund genommen zu werden, um zum Spülen der Nase Atemluft in den Behälterteil 12 einzublasen, wobei das Ventil 32 von der Ruhestellung in eine Offenstellung verbringbar ist, in welcher das Ventilglied 36 vom Ventilsitz 34 abgehoben ist.

Das Mundstück 44 weist ein elastisches Material auf, so dass durch Biegen des Mundstücks 44 unterschiedliche Abstände zwischen Mund und Nase des Benutzers bei der Anwendung kompensierbar sind.

In vorliegendem Ausführungsbeispiel ist das Mundstück 44 abgewinkelt ausgebildet. Es ist jedoch auch denkbar, dass das Mundstück 44 gerade ausgebildet ist. In diesem Fall verläuft die Aufnahme 42 für das Mundstück 44 etwa radial.

Wenn die erfindungsgemässe Nasendusche 10 mit Spülflüssigkeit 14 gefüllt ist und wie in Fig. 1 gezeigt von dem Kappenteil 24 verschlossen sowie um 180° gekippt ist (d.h. in Überkopflage), wirkt der hydrostatische Druck der Spülflüssigkeit 14 auf das tellerförmige Ventilglied 36 des Ventils 32, so dass keine Spülflüssigkeit 14 vom Behälterteil 12 in einen Einblasluftkanal 68 des Mundstücks 44 gelangen kann. Wegen des verengten Flaschenhalses 20 und dem verengten hohlzylinderförmigen Durchgang 50 über den Ausströmungskanal 60 zur Austrittsöffnung 64, kann über den Nasenansatz 62 von aussen keine Umgebungsluft in den Behälter gelangen, so dass die Spülflüssigkeit 14 vom Behälterteil 12 nicht zur Austrittsöffnung gelangen kann. Um die Spülflüssigkeit 14 aus dem Behälterteil 12 abfliessen lassen zu können, muss im Behälterteil 12 ein Druckunterschied hervorgerufen werden.

Wie dies erfindungsgemäss erfolgt, wird im Folgenden im Zusammenhang mit Fig. 2 im Detail erläutert.

Bei Ansetzen des Nasenansatzes 62 der erfindungsgemässen Nasendusche 10 an ein erstes Nasenloch 70 des Benutzers und Umschliessen des Mundstücks 44 der erfindungsgemässen Nasendusche 10 mit den Lippen 72 des Benutzers sowie Einblasen von Atemluft in das Mundstück 44, kommt es zu einem Druckanstieg durch die Atemluft im Einblasluftkanal 68 des Mundstücks 44.

Da das tellerförmige Ventilglied 36 den Weg der Atemluft in den Behälterteil 12 versperrt, muss der beaufschlagte Atemdruck erst die Schliesskraft des Ventilgliedes 36 überwinden. Mit anderen Worten, der Atemluftdruck muss grösser als die Summe der Schliesskraft des Ventilgliedes 36 selbst plus des hydrostatischen Drucks der Spülflüssigkeit 14 sein, damit das Ventilglied 36 sich vom Ventilsitz 34 abheben kann, um Atemluft in den Behälterteil 12 zu bringen und so einen Druckunterschied im Behälterteil 12 zu erzeugen.

Während des Druckanstiegs der Atemluft im Einblasluftkanal 68 des Mundstücks 44 kommt ein weiterer Effekt bei der vorliegenden Erfindung zum Tragen. Als Voraussetzung für den Druckanstieg legt sich das Gaumensegel 76 des Benutzers dicht an eine Rachenhinterwand 78 und trennt so einen Nasenraum 80 von einem Mund-Rachenraum 88 ab. Der Nasenraum 80 ist gebildet von einer linken Nasenhöhle 82 und einer rechten Nasenhöhle 86, wobei diese durch eine Nasenscheidewand 84 räumlich voneinander getrennt sind und hinter der Nasenscheidewand 84 in einen gemeinsamen Nasen-Rachenraum 81 übergehen. Nach unten sind die Nasenhöhlen 82, 86 durch einen harten Gaumen 89 und der Nasen-Rachenraum 81 durch das Gaumensegel 76 begrenzt.

Folglich kann die Spülflüssigkeit 14 aus der erfindungsgemässen Nasendusche 10, beim Einblasen der Atemluft durch das Mundstück 44 in den Behälterteil 12, ausschliesslich durch den Nasenraum 80 und das andere Nasenloch 92 abfliessen und nicht, wie bei bekannten Nasenduschen, nach unten in den Mund-Rachenraum 88 gelangen.

Entsprechend fliesst die Spülflüssigkeit 14 bei Beaufschlagung des Ventils 32 mit Atemluft aus dem Mundstück 44 und Überwindung der Schliesskraft des Ventilglieds 36 vom Behälterteil 12 durch den Durchgang 50 sowie den Ausströmungskanal 60 zur Austrittsöffnung 64 des Nasenansatzes 62 und von dort über das (hier) linke Nasenloch 92 in die (hier) linke Nasenhöhle 86, dort zur Hinterkante 90 der Nasenscheidewand 84 und dann in umgekehrter Flussrichtung durch die (hier) rechte Nasenhöhle 82 zum (hier) rechten Nasenloch 70, wo die Spülflüssigkeit 14 dann wieder austritt. Ein Verschlucken der Spülflüssigkeit 14 ist somit mit der erfindungsgemässen Nasendusche 10 nicht mehr möglich, da das Gaumensegel 78 den Weg zum Mund-Rachenraum 88 vollständig verschliesst.

Wird mit dem Einblasen von Atemluft durch das Mundstück 44 aufgehört, gibt das Gaumensegel 76 den Weg vom Nasenraum 80 in den Mund-Rachenraum 88 frei, jedoch schliesst das Ventil 32, wodurch das Ausfliessen von weiterer Spülflüssigkeit 14 unterbrochen ist.

An dieser Stelle sei erwähnt, dass der Kappenteil 24 anatomisch derart geformt ist, dass die Nasendusche 10 mit einer Hand gehalten werden kann und derart am ersten Nasenloch 70 positionierbar ist, dass aufgrund des hydrostatischen Drucks auf die Spülflüssigkeit 14 und das Ventilglied 36 ein vorzeitiges Auslaufen der Spülflüssigkeit 14 aus dem Behälterteil 12 unterbunden ist.

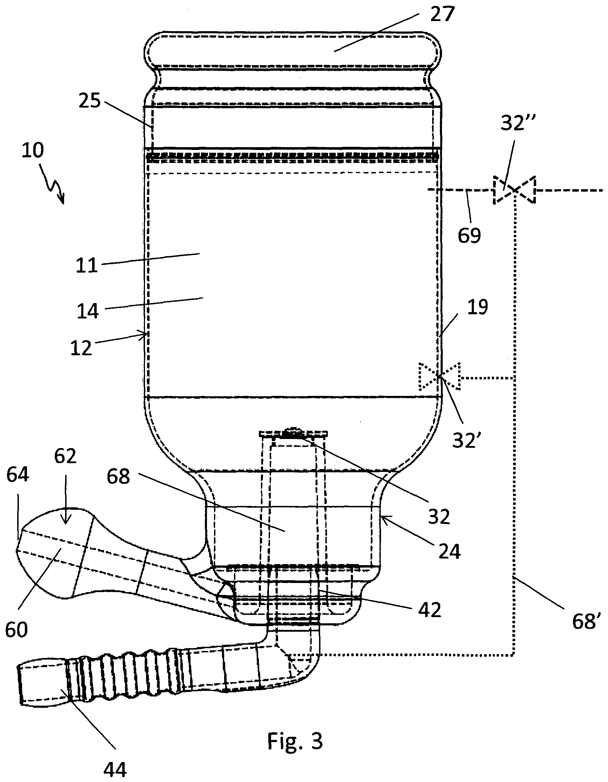

Bei einem weiteren Ausführungsbeispiel der vorliegenden Erfindung gemäss Fig. 3 ist der Behälterteil 12 einstückig mit dem Kappenteil 24 ausgebildet. Es werden die gleichen Bezugszeichen wie im Zusammenhang mit Fig. 1 und Fig. 2 verwendet.

Da der Behälterteil 12 und der Kappenteil 24 zusammen einstückig ausgebildet sind, ist zum Befüllen des Behälterteils 12 mit Spülflüssigkeit 14 eine Nachfüllöffnung 25 im Behälterteil 12 vorgesehen, welche mittels eines Deckels 27 verschliessbar ist.

Das Ventil 32 ist in gleicher Art und Weise dem Kappenteil 24 zugeordnet und auch das Mundstück 44 ist in gleicher Art und Weise in die Aufnahme 42 des Kappenteils 24 eingesteckt, wie im Zusammenhang mit Fig. 1 im Detail erläutert.

Entsprechend ist die funktionale Wirkung beim Ausführungsbeispiel gemäss Fig. 3 identisch mit jener wie im Zusammenhang mit Fig. 2 im Detail erläutert.

Folglich ist das Ventil 32 in der Ruhestellung geschlossen, so dass das Abfliessen von Spülflüssigkeit 14 aus dem Behälterteil 12 in Richtung zur Austrittsöffnung 64 unterbunden ist und das Ventil 32 ist durch Beaufschlagung mit Atemluft, in dem der Benutzer in das Mundstück 44 Atemluft einbläst, in die Offenstellung verbringbar, so dass in der Offenstellung des Ventils 32 Atemluft in den Behälterteil 12 gelangt, wodurch das Abfliessen von Spülflüssigkeit 14 aus dem Behälterteil 12 in Richtung zur Austrittsöffnung 64 freigegeben ist.

Alternativ sei an dieser Stelle erwähnt, dass das Mundstück 44 auch über eine Schlauchverbindung 68' (gestrichelt dargestellt) direkt mit einem an einer Aussenwand 19 des Behälterteils 12 angebrachten Ventil 32' verbindbar ist, wie in Fig. 3 gestrichelt angedeutet. Dabei ist das Ventil 32' in einer Öffnung in der Aussenwand 19 des Behälterteils 12 eingesetzt, mit der gleichen funktionalen Wirkung wie im Zusammenhang mit Fig. 2 im Detail beschrieben. Es sei insbesondere erwähnt, dass bei Offenstellung des Ventils 32' ebenfalls Atemluft aus dem Mundstück 44 in den Behälterteil 12 gelangt und so das Abfliessen von Spülflüssigkeit 14 aus dem Behälterteil 12 in Richtung zur Austrittsöffnung 64 freigegeben ist.

Des Weiteren ist es auch möglich, dass das Ventil 32'' wie in Fig. 3, ebenfalls gestrichelt gezeichnet, sich in einem Einlasskanal 69 befindet, wobei das Ventil 32'' in Ruhestellung den Zugang von Umgebungsluft in das Innere 11 des Behälterteils 12 unterbindet und bei Beaufschlagung des Ventils 32'' mit Atemluft, indem der Benutzer in das Mundstück 44 Atemluft einbläst, in die Offenstellung verbringbar ist, so dass in der Offenstellung des Ventils 32'' Umgebungsluft in den Behälterteil 12 gelangt, wodurch das Abfliessen von Spülflüssigkeit 14 aus dem Behälterteil 12 in Richtung zur Austrittsöffnung 64 freigegeben ist.

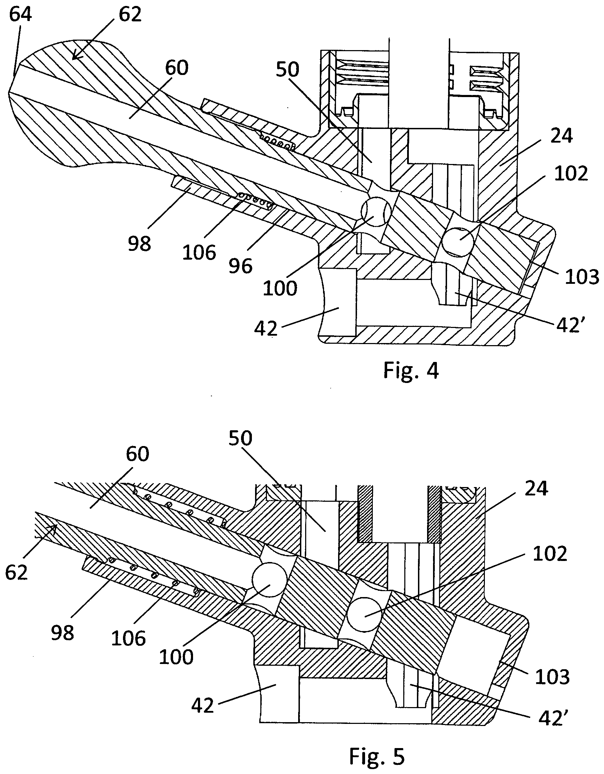

Bei der Ausführungsform gemäss Fig. 4 und 5 ist der Nasenansatz 62 nicht mit dem Kappenteil 24 integral ausgebildet, sondern er ist ein separates, geradliniges Teil, welches in einer sacklochförmigen Aufnahme 96 in seiner Längsrichtung verschiebbar gelagert ist. Die Aufnahme 96 ist durch den Kappenteil 24 und einen daran integral angeformten Fortsatz 98 ausgebildet.

Der Fortsatz 98 ist in Richtung gegen den Flaschenboden 16 abgewinkelt.

Im Nasenansatz 62 ist der Ausströmungskanal 60 sacklochartig ausgebildet, wobei beim geschlossenen Ende des Ausströmungskanals 60 innen, kreuzweise radiale erste Durchlässe 100 durch den Nasenansatz 62 mit dem Ausströmungskanal 60 verbunden sind.

Am Nasenansatz 62 sind kreuzweise radiale zweite Durchlässe 102 ausgenommen, welche vom Ausströmungskanal 60 getrennt sind.

Eine zwischen dem Fortsatz 98 und dem Nasenansatz 62 wirkende Feder 106 drängt den Nasenansatz 62 in eine in Fig. 5 gezeigte (ausgefahrene) Ruhestellung, in welcher die ersten und zweiten Durchlässe 100, 102 durch den Kappenteil 24 verschlossen sind.

Gleichzeitig verschliesst der Nasenansatz 62 einen Verbindungskanal 42' zwischen der Aufnahme 42 des Kappenteils 24 für das Mundstück 44 und dem Behälterteil 12.

Zum Spülen des Nasenraums 80 wird der Nasenansatz 62 an das eine Nasenloch 70 angesetzt und durch Drücken des Kappenteils 24 in Richtung gegen die Nase wird der Nasenansatz 62, gegen die Kraft der Feder 106, in Richtung des Kappenteils 24 hinein verschoben, bis der Nasenansatz 62 am Boden 103 der Aufnahme 96 anliegt, wie dies Fig. 4 zeigt.

Von der Umgebung führt ein Zufuhrloch den Kappenteil 24 zum Boden 103 der Aufnahme 96.

In der in Fig. 4 gezeigten Arbeits- bzw. Spülstellung geben einerseits die zweiten Durchlässe 102 den Verbindungskanal 42' frei und stellt andererseits für die ersten Durchlässe 100 die Strömungsverbindung zwischen dem Durchgang 50 und dem Ausströmungskanal 60 her.

Wird durch das in die Aufnahme 42 eingesteckte Mundstück (nicht gezeigt) Atemluft in den Behälterteil 12 eingeblasen, tritt Spülflüssigkeit 14 durch die ersten Durchlässe 100 und den Ausströmungskanal 60 aus und gelangt so in die Nase des Benutzers.

Wird der Kappenteil 24 von der Nase wegbewegt, bewegt sich der als doppeltes Ventilglied wirkende Nasenansatz 62 unter der Kraft der Feder 106 in die Ruhestellung zurück.

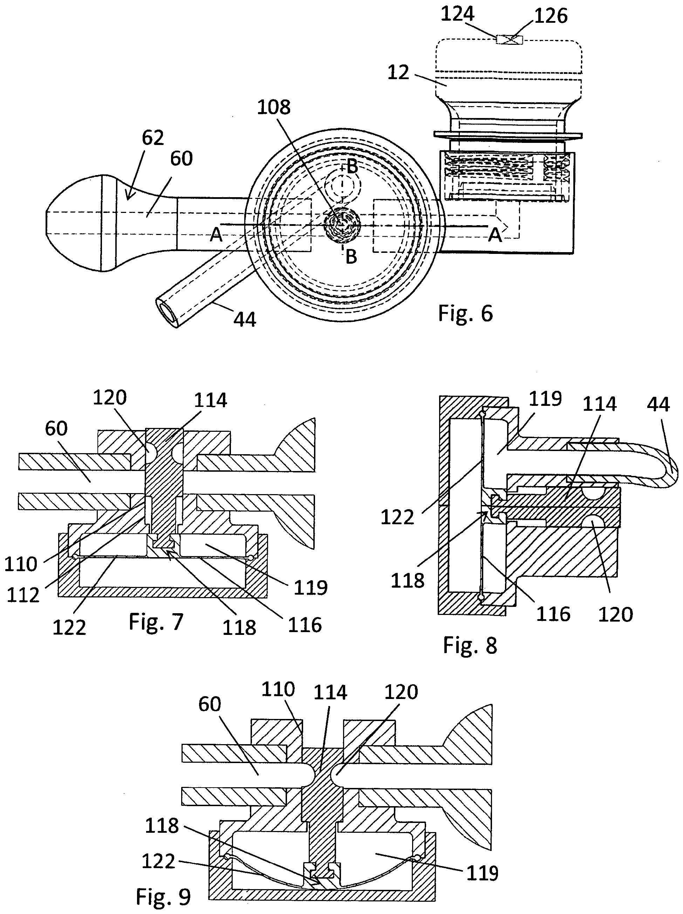

Fig. 6 in Verbindung mit Fig. 7 zeigt eine weitere Ausführungsform der vorliegenden Erfindung, bei der das Ventil 108 den Ausströmungskanal 60 im Nasenansatz 62 verschliesst und bei Beaufschlagung mit Atemluft frei gibt.

Der Nasenansatz 62 weist einen Durchgang 110 quer zum Ausströmungskanal 60 als Ventilsitz 112 auf. Der Ventilsitz 112 wirkt mit einem als Verschlussstift 114 ausgebildeten und mit einem Betätigungsorgan 116 zusammenwirkenden Ventilglied 118 zusammen.

In der Ruhestellung gemäss Fig. 6 verschliesst der Verschlussstift 114 den Ausströmungskanal 60.

Bei Beaufschlagung eines Einblasraumes 119 zwischen dem Betätigungsorgan 116 und dem Mundstück 44 mit Atemluft aus dem Mundstück 44, wie in Fig. 8 in Verbindung mit Fig. 9 gezeigt, verschiebt das Betätigungsorgan 116 den Verschlussstift 114 derart, dass ein Durchlass 120 in Form einer Umfangsnut im Verschlussstift 114 den Ausströmungskanal 60 freigibt.

Das Betätigungsorgan 116 weist vorzugsweise eine gummielastische Membran 122 auf, die sich in Abhängigkeit von der Atemluft im Einblasraum 119, wie in Fig. 9 gezeigt ausdehnen kann, oder wie in Fig. 7 gezeigt wieder zusammenziehen kann, um den Verschlussstift 114 im Durchgang 110 entsprechend hin und her zu bewegen.

Vorzugsweise führt vom Einblasraum 119 ein Verbindungskanal (nicht gezeigt) mit kleinerem Querschnitt als der Ausströmungskanal 60 zum Inneren 11 des Behälterteils 12, um die Atemluft bei Offenstellung des Ventils 108 vom Mundstück 44 in den Behälterteil 12 zu führen.

Es ist aber auch denkbar, dass statt des Verbindungskanals der Behälterteil 12 eine Belüftungsöffnung 124 aufweist und mit einem Rückschlagventil 126 versehen ist, welches das Austreten von Spülflüssigkeit 14 aus dem Behälterteil 12 durch die Belüftungsöffnung 124 verhindert und das Einströmen von Umgebungsluft durch die Belüftungsöffnung 124 in den Behälterteil 12 zulässt, so dass in Offenstellung des Ventils 108 die in den Behälterteil 12 einströmende Umgebungsluft das Abfliessen der Spülflüssigkeit 14 in Richtung des Ausströmungskanals 60 ermöglicht.

## Patentansprüche

1. Nasendusche aufweisend:
einen Behälterteil (12) und einen Kappenteil (24), wobei der Behälterteil (12) zur Aufnahme von Spülflüssigkeit (14) dient und den Kappenteil (24) trägt;
einen Nasenansatz (62) des Kappenteils (24) mit einer über einen Ausströmungskanal (60) mit einem Inneren (11) des Behälterteils (12) strömungsverbundenen Austrittsöffnung (64) für die Spülflüssigkeit (14), wobei der Nasenansatz (62) dazu bestimmt ist an ein Nasenloch (70, 92) des Benutzers angesetzt zu werden; und
ein Ventil (32, 32', 32'', 108);
**dadurch gekennzeichnet, dass**
dem Behälterteil (12) oder dem Kappenteil (24) ein Mundstück (44) zur Steuerung des Ventils (32, 32', 32'', 108) zugeordnet ist;
das Ventil (32, 32', 32'', 108) in einer Ruhestellung geschlossen und dadurch das Ausfliessen von Spülflüssigkeit (14) durch den Nasenansatz (62) unterbunden ist; und
das Ventil (32, 32', 32'', 108) durch Beaufschlagung mit Atemluft, indem der Benutzer in das Mundstück (44) Atemluft einbläst, in eine Offenstellung verbringbar ist und dadurch Spülflüssigkeit (14) durch den Nasenansatz (62) ausfliesst, wobei zum Ausfliessen Atemluft oder Umgebungsluft in den Behälterteil (12) einströmt.

2. Nasendusche nach Anspruch 1, **dadurch gekennzeichnet, dass** der Behälterteil (12) als eigenständiges Bauteil und der Kappenteil (24) als eigenständiges Bauteil ausgebildet sind, und diese vorzugsweise trennbar aneinander befestigt sind.

3. Nasendusche nach Anspruch 2, **dadurch gekennzeichnet, dass** der Kappenteil (24) und der Behälterteil (12) mittels einer Schraubverbindung oder eines Bajonettverschlusses aneinander befestigt sind.

4. Nasendusche nach Anspruch 2, **dadurch gekennzeichnet, dass** der Kappenteil (24) und der Behälterteil (12) mittels eines Presssitzes aneinander befestigt sind.

5. Nasendusche nach Anspruch 1, **dadurch gekennzeichnet, dass** der Behälterteil (12) einstückig mit dem Kappenteil (24) ausgebildet ist.

6. Nasendusche nach Anspruch 5, **dadurch gekennzeichnet, dass** der Behälterteil (12) eine Nachfüllöffnung (25) aufweist, welche mittels eines Deckels (27) verschliessbar ist.

7. Nasendusche nach einem der Ansprüche 1 bis 6, **dadurch gekennzeichnet, dass** das Ventil (32, 32', 32'') in Ruhestellung einen vom Mundstück (44) zum Behälterteil (12) führenden Einblasluftkanal (68, 68') verschliesst, wodurch keine Atemluft oder Umgebungsluft in den Behälterteil (12) gelangt und das Abfliessen von Spülflüssigkeit (14) aus dem Behälterteil (12) in Richtung zur Austrittsöffnung (64) unterbunden ist, und das Ventil (32, 32', 32'') bei Beaufschlagung mit Atemluft in Offenstellung verbringbar ist, so dass die Atemluft über den Einblasluftkanal (68, 68') oder Umgebungsluft in den Behälterteil (12) gelangt und die Spülflüssigkeit (14) aus dem Behälterteil (12) in Richtung zur Austrittsöffnung (64) abfliessen kann.

8. Nasendusche nach einem der Ansprüche 1 bis 7, **dadurch gekennzeichnet, dass** das Ventil (32) einen vorzugsweise an dem Kappenteil (24) angeformten, vorzugsweise ringförmigen Ventilsitz (34) und ein mit diesem zusammenwirkendes vorzugsweise tellerförmiges Ventilglied (36) aufweist.

9. Nasendusche nach Anspruch 8, **gekennzeichnet durch** einen Steg (52) mit einem vorzugsweise wenigstens annährend eiförmig oder kugelförmig verdickten Kopf (54), wobei das Ventilglied (36) vorzugsweise unter Vorspannung am Ventilsitz (34) anliegt sowie membranartig, elastisch verformbar ausgebildet ist, zentrisch ein Durchgangsloch (56) aufweist, bei der Montage mit dem Durchgangsloch (56) über den Kopf (54) ziehbar ist und im montierten Zustand den Kopf (54) hintergreift.

10. Nasendusche nach einem der Ansprüche 1 bis 9, **dadurch gekennzeichnet, dass** das am Kappenteil (24) angeordnete Mundstück (44) ein elastisches Material aufweist, so dass unterschiedliche Abstände zwischen Mund und Nase des Benutzers bei der Anwendung kompensierbar sind.

11. Nasendusche nach einem der Ansprüche 1 bis 10, **dadurch gekennzeichnet, dass** das Mundstück (44) austauschbar ist und vorzugsweise in verschiedenen Dimensionen für physiognomisch unterschiedliche Anwendergruppen vorliegt.

12. Nasendusche nach einem der Ansprüche 1 bis 11, **dadurch gekennzeichnet, dass** das Mundstück (44) auswechselbar in eine Aufnahme (42) des Kappenteils (24) einsteckbar oder auf einen Ansatz des Kappenteils (24) aufsteckbar ist, wobei das Mundstück (44) vorzugsweise gerade oder abgewinkelt ausgebildet ist.

13. Nasendusche nach Anspruch 1, **dadurch gekennzeichnet, dass** das Ventil (108) in Ruhestellung den Ausströmungskanal (60) verschliesst und in Offenstellung den Ausströmungskanal (60) freigibt.

14. Nasendusche nach Anspruch 13, **dadurch gekennzeichnet, dass** das Ventil (108) einen Durchgang (110) quer zum Ausströmungskanal (60) als Ventilsitz (112) aufweist, welcher mit einem als Verschlussstift (114) ausgebildeten und mit einem Betätigungsorgan (116) zusammenwirkenden Ventilglied derart zusammenwirkt, dass in Ruhestellung der Verschlussstift (114) den Ausströmungskanal (60) verschliesst und in Offenstellung, bei Beaufschlagung mit Atemluft, das Betätigungsorgan (116) den Verschlussstift (114) derart verschiebt, dass der Durchlass (120) den Ausströmungskanal (60) freigibt.

15. Nasendusche nach Anspruch 13 oder 14, **dadurch gekennzeichnet, dass** der Behälterteil (12) eine Belüftungsöffnung (124) aufweist und vorzugsweise mit einem Rückschlagventil (126) versehen ist, welches das Austreten von Spülflüssigkeit (14) aus dem Behälterteil (12) durch die Belüftungsöffnung (124) verhindert und das Einströmen von Umgebungsluft durch die Belüftungsöffnung (124) in den Behälterteil (12) zulässt.

## Claims

1. A nasal douche, comprising:
a container part (12) and a cap part (24), wherein the container part (12) serves to receive irrigation liquid (14) and carries the cap part (24);
a nose attachment (62) of the cap part (24) with an outlet opening (64) for the irrigation liquid (14) flow-connected to an interior (11) of the container part (12) via a discharge channel (60), the nose attachment (62) being intended for attachment to a nostril (70, 92) of the user; and
a valve (32, 32', 32", 108);
**characterized in that**
a mouthpiece (44) for controlling the valve (32, 32', 32", 108) is assigned to the container part (12) or the cap part (24);
the valve (32, 32', 32", 108) is closed in a rest position and this prevents the drainage of irrigation liquid (14) through the nose attachment (62); and
the valve (32, 32', 32", 108) can be brought into an open position by actuation with respiratory air when the user blows respiratory air into the mouthpiece (44) and irrigation liquid (14) flows out through the nose attachment (62) as a result thereof, with respiratory air or ambient air flowing into the container part (12) for drainage.

2. The nasal douche as claimed in claim 1, **characterized in that** the container part (12) is embodied as an independent component and the cap part (24) is embodied as an independent component and these are fastened to one another, preferably in a separable manner.

3. The nasal douche as claimed in claim 2, **characterized in that** the cap part (24) and the container part (12) are fastened to one another by means of a screw-in connection or a bayonet closure.

4. The nasal douche as claimed in claim 2, **characterized in that** the cap part (24) and the container part (12) are fastened to one another by means of a force fit.

5. The nasal douche as claimed in claim 1, **characterized in that** the container part (12) has an integral embodiment with the cap part (24).

6. The nasal douche as claimed in claim 5, **characterized in that** the container part (12) has a refill opening (25), which is sealable by means of a lid (27).

7. The nasal douche as claimed in one of claims 1 to 6, **characterized in that** the valve (32, 32', 32"), in the rest position, seals a blown-in air channel (68, 68') leading from the mouthpiece (44) to the container part (12), as a result of which no respiratory air or ambient air reaches the container part (12) and the drainage of irrigation liquid (14) from the container part (12) in the direction of the outlet opening (64) is prevented, and the valve (32, 32', 32") can be brought into an open position when actuated with respiratory air such that the respiratory air reaches the container part (12) via the blown-in air channel (68, 68') or ambient air reaches the container part (12) and the irrigation liquid (14) can drain from the container part (12) in the direction of the outlet opening (64).

8. The nasal douche as claimed in one of claims 1 to 7, **characterized in that** the valve (32) has a valve seat (34), which is preferably ring-shaped and preferably formed on the cap part (24), and a preferably plate-shaped valve member (36) interacting therewith.

9. The nasal douche as claimed in claim 8, **characterized by** a web (52) with a head (54) which is preferably thickened at least approximately in an ovoid or spherical manner, wherein the valve member (36) abuts on the valve seat (34), preferably under pretension, and it is embodied in a membrane-like, elastically deformable manner, it has a through hole (56) at a central location, it can be pulled over the head (54) with the through hole (56) during assembly and it engages behind the head (54) in the assembled state.

10. The nasal douche as claimed in one of claims 1 to 9, **characterized in that** the mouthpiece (44) arranged at the cap part (24) has an elastic material such that different distances between mouth and nose of the user are compensable during the application.

11. The nasal douche as claimed in one of claims 1 to 10, **characterized in that** the mouthpiece (44) is interchangeable and preferably available with different dimensions for physiognomically different user groups.

12. The nasal douche as claimed in one of claims 1 to 11, **characterized in that** the mouthpiece (44) is insertable in an interchangeable manner into a receptacle (42) of the cap part (24) or pluggable onto an attachment of the cap part (24), wherein the mouthpiece (44) preferably has a straight or angled embodiment.

13. The nasal douche as claimed in claim 1, **characterized in that** the valve (108) seals the discharge channel (60) in the rest position and opens up the discharge channel (60) in the open position.

14. The nasal douche as claimed in claim 13, **characterized in that** the valve (108) has a passage (110) across the discharge channel (60) as a valve seat (112), which interacts with a valve member, which is embodied as a closure pin (114) and interacts with an actuation organ (116), in such a way that the closure pin (114) seals the discharge channel (60) in the rest position and the actuation organ (116) displaces the closure pin (114) in the open position when actuated by respiratory air in such a way that the aperture (120) opens up the discharge channel (60).

15. The nasal douche as claimed in claim 13 or 14, **characterized in that** the container part (12) has a ventilation opening (124) and it is preferably provided with a check valve (126) which prevents the emergence of irrigation liquid (14) from the container part (12) through the ventilation opening (124) and admits the inflow of ambient air through the ventilation opening (124) into the container part (12).

## Revendications

1. Douche nasale présentant:
une partie de conteneur (12) et une partie de capuchon (24), la partie de conteneur (12) servant à recevoir un liquide de rinçage (14) et portant la partie de capuchon (24);
un embout nasal (62) de la partie de capuchon (24) avec une ouverture de sortie (64) pour le liquide de rinçage (14) reliée pour l'écoulement avec un intérieur (11) de la partie de conteneur (12) au moyen d'un canal d'écoulement (60), l'embout nasal (62) étant prévu pour être appliqué à une narine (70, 92) de l'utilisateur; et
une valve (32, 32', 32", 108);
**caractérisée en ce que**
un embout buccal (44) pour la commande de la valve (32, 32', 32", 108) est associé à la partie de conteneur (12) ou à la partie de capuchon (24);
la valve (32, 32', 32", 108) est fermée dans une position de repos et ce faisant l'écoulement de liquide de rinçage (14) à travers l'embout nasal (62) est empêchée; et
la valve (32, 32', 32", 108) peut être amenée dans une position ouverte par activation par de l'air respiré quand l'utilisateur souffle de l'air respiré à travers l'embout buccal (44) et ce faisant du liquide de rinçage (14) s'écoule à travers l'embout nasal (62), de l'air respiré ou de l'air ambiant affluant dans la partie de conteneur (12) pour l'écoulement.

2. Douche nasale selon la revendication 1, **caractérisée en ce que** la partie de conteneur (12) est façonnée comme un composant indépendant et la partie de capuchon (24) est façonnée comme un composant indépendant, et celles-ci sont fixées l'une à l'autre, préférablement de façon séparable.

3. Douche nasale selon la revendication 2, **caractérisée en ce que** la partie de capuchon (24) et la partie de conteneur (12) sont fixées l'une à l'autre au moyen d'un assemblage à vis ou d'une fermeture à baïonnette.

4. Douche nasale selon la revendication 2, **caractérisée en ce que** la partie de capuchon (24) et la partie de conteneur (12) sont fixées l'une à l'autre au moyen d'un ajustage serré.

5. Douche nasale selon la revendication 1, **caractérisée en ce que** la partie de conteneur (12) est façonnée en une pièce avec la partie de capuchon (24).

6. Douche nasale selon la revendication 5, **caractérisée en ce que** la partie de conteneur (12) présente une ouverture de rechargement (25) qui peut être fermée au moyen d'un couvercle (27).

7. Douche nasale selon l'une des revendications 1 à 6, **caractérisée en ce que** la valve (32, 32', 32"), dans la position de repos, ferme un canal d'insufflation d'air (68, 68') menant de l'embout buccal (44) à la partie de conteneur (12), ce qui fait qu'il n'y a pas d'air respiré ou d'air ambiant qui arrive dans la partie de conteneur (12) et que l'écoulement de liquide de rinçage (14) de la partie de conteneur (12) dans la direction de l'ouverture de sortie (64) est empêchée, et la valve (32, 32', 32") peut être amenée dans une position ouverte par activation par de l'air respiré de telle sorte que l'air respiré arrive dans la partie de conteneur (12) au moyen du canal d'insufflation d'air (68, 68') ou de l'air ambiant arrive dans la partie de conteneur (12) et le liquide de rinçage (14) peut s'écouler de la partie de conteneur (12) dans la direction de l'ouverture de sortie (64).

8. Douche nasale selon l'une des revendications 1 à 7, **caractérisée en ce que** la valve (32) présente un siège de valve (34), préférablement façonné sur la partie de capuchon (24) et préférablement en forme d'anneau, et un organe de valve (36), préférablement en forme d'assiette, interagissant avec celui-ci.

9. Douche nasale selon la revendication 8, **caractérisée par** une traverse (52) avec une tête (54) épaissie préférablement au moins approximativement en forme d'oeuf ou en forme de sphère, l'organe de valve (36) étant ajusté au siège de valve (34), préférablement sous précontrainte, étant façonné en forme de membrane et déformable de façon élastique, présentant un trou de passage (56) central, pouvant être tiré au-dessus de la tête (54) avec le trou de passage (56) lors du montage et venant en prise derrière la tête (54) dans l'état monté.

10. Douche nasale selon l'une des revendications 1 à 9, **caractérisée en ce que** l'embout buccal (44) disposé sur la partie de capuchon (24) présente un matériau élastique de telle sorte que différentes distances entre la bouche et le nez de l'utilisateur sont compensables lors de l'utilisation.

11. Douche nasale selon l'une des revendications 1 à 10, **caractérisée en ce que** l'embout buccal (44) est interchangeable et préférablement disponible avec différentes dimensions pour des groupes d'utilisateurs de physiognomie différente.

12. Douche nasale selon l'une des revendications 1 à 11, **caractérisée en ce que** l'embout buccal (44) est emboîtable de façon interchangeable dans un réceptacle (42) de la partie de capuchon (24) ou peut être enfilé sur un embout de la partie de capuchon (24), l'embout buccal (44) étant préférablement façonné droit ou plié.

13. Douche nasale selon la revendication 1, **caractérisée en ce que** la valve (108) ferme le canal d'écoulement (60) dans la position de repos et libère le canal d'écoulement (60) dans la position ouverte.

14. Douche nasale selon la revendication 13, **caractérisée en ce que** la valve (108) présente un passage (110) en travers le canal d'écoulement (60) comme siège de valve (112), lequel interagit avec un organe de valve, qui est façonné comme une tige de fermeture (114) et interagit avec un organe d'actionnement (116), de telle sorte que, dans la position de repos, la tige de fermeture (114) ferme le canal d'écoulement (60) et, dans la position ouverte, l'organe d'actionnement (116) déplace la tige de fermeture (114) lors de l'activation par de l'air respiré de telle sorte que le passage (120) libère le canal d'écoulement (60).

15. Douche nasale selon la revendication 13 ou 14, **caractérisée en ce que** la partie de conteneur (12) présente une ouverture de ventilation (124) et est préférablement munie d'une valve de retenue (126) qui empêche la sortie de liquide de rinçage (14) de la partie de conteneur (12) à travers l'ouverture de ventilation (124) et permet l'afflux d'air ambiant à travers l'ouverture de ventilation (124) dans la partie de conteneur (12).
